# EUROPEAN PATENT APPLICATION

(11) **EP 2 759 300 A2**
(43) Date of publication of application: **30.07.2014**
(21) Application number: 12814911.9
(22) Date of filing: 12.07.2012
(51) Int. Cl.: A61K 33/06, A61K 31/7004, A61K 31/198, A61K 31/718, A61P 7/08

(54) **USE OF A COMPOSITION IN THE PRODUCTION OF A DIALYSIS SOLUTION FOR THE TREATMENT OF CEREBROVASCULAR DISEASES BY MEANS OF PERITONEAL DIALYSIS**

(30) Priority: 21.07.2011 ES 201100829
(71) Applicant: Universidad Complutense De Madrid, 28040 Madrid (ES)
(72) Inventor: MORA SÁNCHEZ, María Ángeles, 28040 Madrid (ES); LIZASOAÍN HERNÁNDEZ, Ignacio, 28040 Madrid (ES); SÁNCHEZ-PRIETO BORJA, José, 28040 Madrid (ES); TORRES MOLINA, Magdalena, 28040 Madrid (ES); GODINO ALARCON, María del Carmen, 28040 Madrid (ES); SOBRADO SANZ, Monica, 28040 Madrid (ES); GONZÁLES ROMERA, Victor Manuel, 28040 Madrid (ES); VIVANCOS MORA, José Aurelio, 28223 Pozuelo de Alarcón (ES)
(74) Representative: Carpintero Lopez, Francisco
(86) International application number: PCT/ES2012/000195
(87) International publication number: WO 2013/011166

(57) **Abstract**

The invention relates to the use of an isotonic composition buffered with lactate/phosphate in a physiologically acceptable pH range (6 - 7.5), with low concentrations of glutamate and a physiological or smaller concentration of glucose, in the production of a peritoneal dialysis solution for the acute treatment of stroke and other cerebrovascular diseases. The application of this dialysis solution in the peritoneal cavity significantly reduces the volume of cerebral infarction associated with the occlusion of the middle cerebral artery and, consequently, the associated neurological damage. The invention is suitable for use in biomedicine in cerebrovascular pathologies, including hemorrhagic or cerebral ischemic stroke.

## Description

### Field of the Invention

The present invention relates to a composition for the preparation of a peritoneal dialysis solution, more specifically, a peritoneal dialysis solution that can be used in the treatment of cerebrovascular diseases, particularly in the treatment of cerebral stroke. The invention is therefore comprised in the biomedical sector and the scope of application is for the preparation of peritoneal dialysis solutions for the treatment of cerebrovascular pathologies and diseases.

### State of the Art

Cerebrovascular diseases (CVDs) or stroke comprise a group of cerebral vasculature disorders that are characterized by reduced blood flow in the brain with the subsequent transient or permanent impairment of brain function at a focal or global level. CVDs are the third cause of death in the developed world; in Spain they are the second cause of death in men and the first cause of death in women. They are considered the first cause of disability and of vascular dementia, affecting 50% of the population over 60 years of age; they are therefore a health problem and a medical emergency today. Cerebrovascular diseases can be hemorrhagic or ischemic considering the type of vascular event.

An ischemic cerebrovascular event occurs when an artery supplying blood to the brain is blocked, abruptly reducing or interrupting blood flow and causing brain infarction over time. About 80% of all cerebrovascular events are ischemic. Thrombi caused by atherosclerosis or emboli caused by blood clotting disorders are the most common cause of arterial blockage and cerebral infarction. Thrombosis is the result of pathological transformations in the arterial tree determining vessel occlusion and flow interruption at that level. They are generally atherosclerotic plaques favoring platelet aggregation, so intraluminal thrombi can lead to occlusion. On the other hand, embolism is the result of materials originating from another point of the bloodstream and of unwanted clotting processes. The clotting process is necessary and beneficial throughout the body as it stops bleeding and allows repairing damaged areas of the arteries or veins. However, when blood clots are formed in the wrong place in an artery, they cause a devastating lesion by interfering with normal blood flow. Clotting problems become more common as a person ages. On the other hand, hemorrhagic strokes are the result of a ruptured blood vessel or an abnormal vascular structure and represent about 20% of the remaining cerebrovascular events. Some hemorrhages develop within the areas of ischemia, which is called "hemorrhagic transformation".

Despite the high incidence of stroke, therapeutic interventions are extremely limited and the only effective treatment today for non-hemorrhagic ischemic stroke is the administration of thrombolytic therapy with tissue plasminogen activator (t-PA, alteplase) to restore blood flow. However, this treatment is only applicable in 3-5% of patients. The following steps are fundamental before administering this compound:
- Thrombolytics must be administered within (but not later than) 4.5 hours after the stroke so that they may have a beneficial effect (Hacke et al., New Eng J Med. 2008. 359: 1317-1329). Unfortunately, most stroke patients reach the hospital more than 4.5 hours after the attack, and therefore treatment cannot be administered.
- Before administering t-PA, it must be confirmed by means of a CAT (computed axial tomography) scan that the stroke is non-hemorrhagic since treatment with t- PA entails the risk of bleeding.
- Some patients are at higher risk of hemorrhaging when these drugs are administered. These include patients with the following conditions: patients being treated with antiplatelet drugs (e.g., aspirin) and/or anticoagulant drugs, patients with clotting disorders, patients with a recent history of bleeding ulcers or atrial fibrillation.

An objective in CVD treatment is to limit neuronal damage (neuroprotection) through drugs or other therapeutic approaches intervening in the ischemic cascade and reducing the amount of damaged tissue. A better clinical result could therefore be obtained, and this translates not only into survival, but also into a better quality of life for patients suffering acute vascular events.

Animal models of cerebral ischemia have contributed to the development of knowledge relating to this problem. Many neuroprotective agents have been tested in these models; however, up until now, none meets the efficacy and safety criteria in clinical trials. In the *Neuroprotection for ischemic stroke: Past, present and future* review (Ginsberg MD. Neuropharmacology. 2008. 55:363-389), the author makes a compilation of neuroprotective agents that have been evaluated both in preclinical studies and in clinical trials for the treatment of ischemic stroke. There is a large variety and amount of agents, or groups of agents, the effects of which have been proven in recent years, and these include: calcium antagonists, glutamate antagonists, GABA agonists, antioxidants or radical scavengers, phospholipid precursors, nitric oxide-dependent signal transduction regulators, leukocyte inhibitors, hemodilution, and other more recent therapies such as hypothermia, high doses of human albumin, magnesium or combined therapies.

### Description of the Invention

The present invention relates to the use of an isotonic buffered composition in the preparation of a dialysis solution for reducing the cerebral infarct volume in cerebrovascular diseases by means of peritoneal dialysis. The composition of the present invention comprises electrolytes, physiological pH-regulating substances and glucose concentrations equal to or less than physiological concentrations of blood plasma. Furthermore, this composition must not contain a glutamate concentration greater than 200 µM. The use of a peritoneal dialysis solution prepared with this composition very significantly reduces (43.1±10.0% reduction) the cerebral infarct volume after middle cerebral artery occlusion (MCAO).

The electrolyte concentration can vary in the physiological range of plasma electrolyte concentrations, always maintaining isotonicity with respect to blood plasma. Blood pressure must be very carefully regulated after a stroke to prevent both hemorrhages due to high blood pressure and hypoperfusion due to low blood pressure, so it is necessary for the composition of the invention to be isotonic.

This specification includes several examples of compositions with different glucose, glutamate or buffering substance concentrations in order to offer evidence of the possible effective combinations in the treatment of stroke, without these examples limiting the scope of the invention. The provided examples must be understood as models used to represent the wide variety of liquids that can be used. As an example, a composition suitable for the preparation of a dialysis solution would be that which contains 110 to 150 mEq/L of sodium ion, 0 to 5 mEq/L of potassium ion, 0 to 2 mEq/L of magnesium ion, 0 to 6 mEq of calcium ion, 80 to 150 mEq/L of chloride ion, 0.5-2 g/L of glucose, 0 to 200 µM of glutamate, respecting isotonicity with respect to blood plasma and maintaining a physiologically acceptable pH.

The pH of the composition of the invention must be physiologically acceptable. In this specification, the term "physiologically acceptable" means that the pH is in the range of 6 to 7.5. The composition furthermore contains a physiological regulatory substance for adjusting the pH in order to keep the dialysis solution buffered. In terms of physiological pH-regulating substances, the composition of the present invention may contain up to 3.5 mmol/L of lactate and must contain 10 mmol/L of phosphate.

Since peritoneal dialysis involves metabolite loss, the composition for the preparation of the dialysis solution can contain different glucose concentrations (0.5-2.0 g/L), the upper limit being the physiological plasma glucose concentration to prevent an increase in plasma glucose concentration due to glucose diffusion from the dialysis liquid into plasma given that hyperglycemia is a poor prognosis factor after a stroke.

Likewise, glucose can be partially substituted with a dextrin which will also contribute to the isotonicity of the dialysis solution.

Any composition meeting these requirements (isotonicity, low glucose concentration, glutamate concentration of less than 200 µM, phosphate-buffered pH) can be used in the preparation of a sterile peritoneal dialysis solution for use in the treatment of cerebrovascular diseases by means of peritoneal dialysis.

This specification describes examples of using the dialysis solution resulting from the composition of the invention used in peritoneal dialysis in rats in which middle cerebral artery occlusion was performed, thus causing cerebral infarction. The infarct volume was significantly less in treated animals (12.17±1.75% - average of all the values obtained in the tests in which the composition of Table 1 was used-) than in the untreated control group (21.42±1.48%), from which it can be deduced that the composition of the invention and its resulting application are effective in reducing ischemic stroke-associated brain damage.

Peritoneal dialysis is one of the treatment options available as an alternative to kidney dialysis for removing waste products and excess liquid from blood when the kidneys stops working properly. The good results obtained in this invention in models of cerebrovascular disease may be due to a similar action, removing a waste product that reaches the blood after a stroke or other cerebrovascular disease. In this sense, some studies on the effects produced by these diseases have been able to attribute a fundamental role in neuronal damage progression to glutamate accumulation in the extracellular medium of the brain parenchyma, hence the most recent research is aimed at scavenging or eliminating blood glutamate. In this context, glutamate-oxaloacetate transaminase (GOT) and glutamate-pyruvate transaminase (GPT) have been used together in treatment using pyruvate or glutamate (WO2004012762). Patent application WO2007105203 describes the use of agents capable of modulating stress hormone activity, particularly adrenergic agonists and antagonists, with or without glutamate modifying enzymes (transaminases, dehydrogenases, decarboxylases, transferases, etc.). Oxaloacetate has also been tested more recently. Oxaloacetate has been proven to stimulate resident glutamate-oxaloacetate transaminase which gives rise to the reduction in plasma glutamate concentration due to the stimulation of the degradation capacity of plasma for this amino acid. It has been demonstrated that this method provides neuroprotection in an animal model (rat) of focal cerebral ischemia (Nagy D, et al. Cell Mol Neurobiol. 2009. 29:827-35) and of head injury (Zlotnik A, et al. J Neurosurg Anesthesiol. 2009. 21:235-41).

To check if the mechanism of action of the dialysis solution prepared with the composition of the invention was related to these findings, the influence of the presence of different glutamate concentrations in said composition has been analyzed and the present specification describes examples in which it has been found that the lower the glutamate concentration the greater the beneficial effect, such that this composition preferably must not contain glutamate concentrations greater than 200 µM, and even more preferably it must not contain glutamate.

In a first aspect, the present invention relates to an isotonic composition with a low glucose concentration, a glutamate concentration equal to or less than 200 µM, preferably equal to zero, and pH buffered with a mixture of lactate and phosphate, for use in the treatment of cerebrovascular diseases by means of peritoneal dialysis.

A second aspect of this invention relates to the use of an isotonic composition with a low glucose concentration, a glutamate concentration equal to or less than 200 µM, preferably equal to zero, and pH buffered with a mixture of lactate and phosphate, in the preparation of a sterile dialysis solution that can be applied in the treatment of a cerebrovascular disease by means of peritoneal dialysis.

As mentioned above, as it is used herein, the term "cerebrovascular disease or CVD" refers to a group of cerebral vasculature disorders that are characterized by reduced blood flow in the brain with the subsequent transient or permanent impairment of brain function at a focal or global level. Cerebrovascular diseases include transient ischemic attack, traumatic brain injury, cerebral ischemic stroke and cerebral hemorrhagic stroke.

The invention also relates to a peritoneal dialysis method for treating cerebrovascular diseases by means of using a sterile dialysis solution prepared with an isotonic composition with a low glucose concentration, a glutamate concentration equal to or less than 200 µM, preferably equal to zero, and pH buffered with a mixture of lactate and phosphate.

For stroke experts, the advantages of this invention and its application are: (1) Usefulness in all types of stroke. (2) Quick application of the technique, even in the ambulance, as soon as stroke code activation takes place. (3) The peritoneal dialysis technique is already successfully applied in other pathologies and peritoneal dialysis solutions are known and in use. (4) The patient does not need to be given any drug. (5) Sophisticated equipment is not required.

### Brief Description of the Drawings

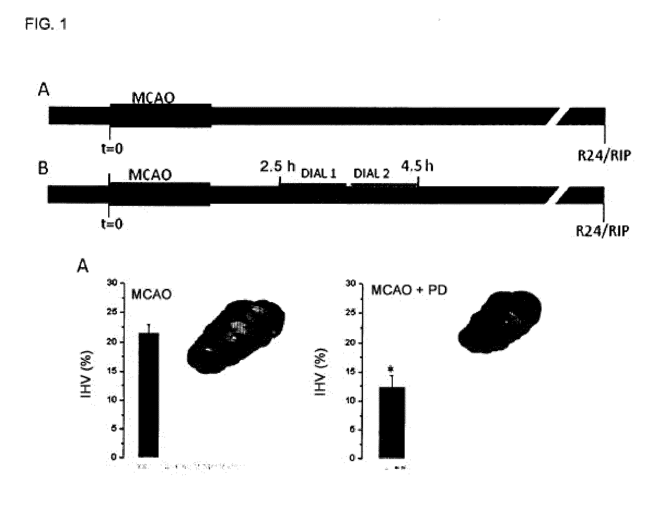

Figure 1 shows the effect of peritoneal dialysis with a dialysis solution prepared from the composition specified in Table 1 on infarct volume after middle cerebral artery occlusion (MCAO).
   A) Mean infarct volume in the group of animals exposed to permanent MCAO for 24 hours (the data is expressed as mean±SEM, n=8). B) Mean infarct volume in the group of animals exposed to permanent MCAO for 24 hours and subjected to two dialysis cycles (the data is expressed as mean±SEM, n=12, ^{*}p<0.001 with respect to the control). t=0 indicates the start of the middle cerebral artery occlusion surgery.

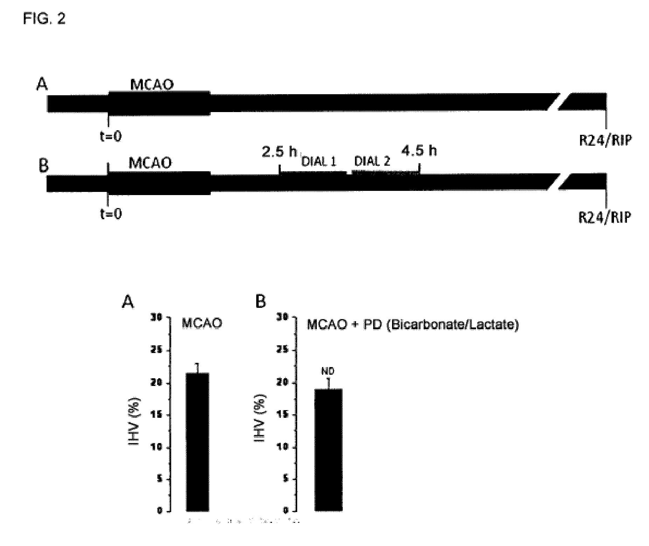

Figure 2 shows the effect of peritoneal dialysis with a dialysis solution prepared from the composition specified in Table 2 on infarct volume after middle cerebral artery occlusion (MCAO).
   A) Mean infarct volume in the group of animals exposed to permanent MCAO for 24 hours (the data is expressed as mean±SEM, n=8). B) Mean infarct volume in the group of animals exposed to permanent MCAO for 24 hours and subjected to two dialysis cycles (the data is expressed as mean±SEM, n=4, ^{*}p>0.05 with respect to the control). t=0 indicates the start of the middle cerebral artery occlusion surgery.

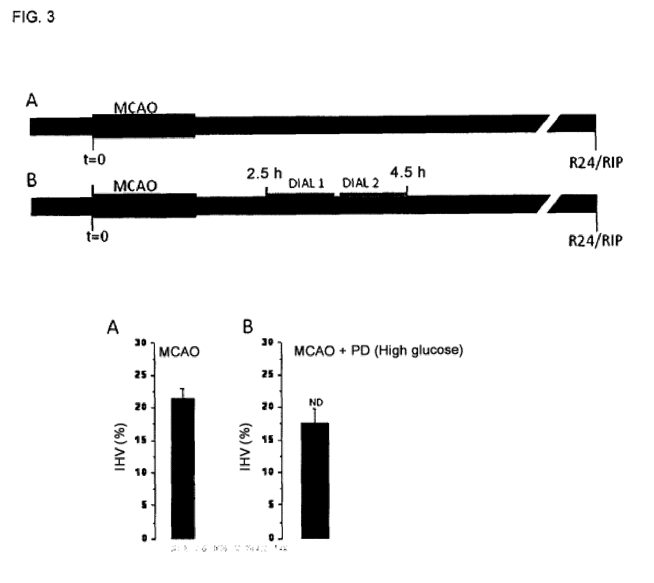

Figure 3 shows the effect of peritoneal dialysis with a dialysis solution prepared from the composition specified in Table 3 on infarct volume after middle cerebral artery occlusion (MCAO).
   A) Mean infarct volume in the group of animals exposed to permanent MCAO for 24 hours (the data is expressed as mean±SEM, n=8). B) Mean infarct volume in the group of animals exposed to permanent MCAO for 24 hours and subjected to two dialysis cycles (the data is expressed as mean±SEM, n=4, ^{*}p>0.05 with respect to the control). t=0 indicates the start of the middle cerebral artery occlusion surgery.

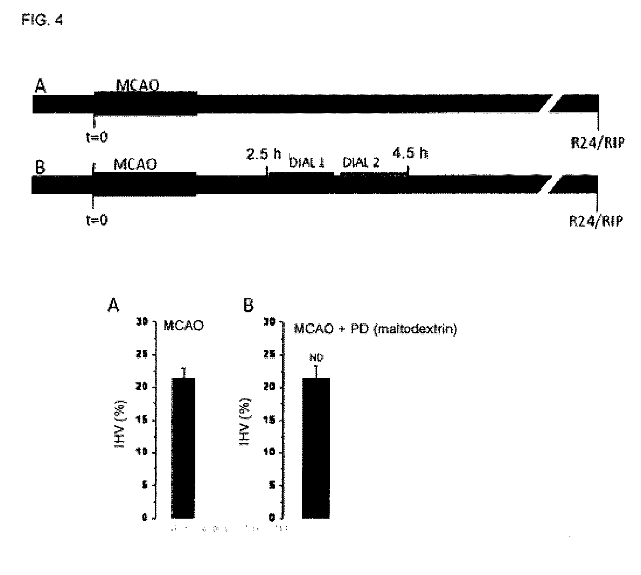

Figure 4 shows the effect of peritoneal dialysis with a dialysis solution prepared from the composition specified in Table 4 on infarct volume after middle cerebral artery occlusion (MCAO).
   A) Mean infarct volume in the group of animals exposed to permanent MCAO for 24 hours (the data is expressed as mean±SEM, n=8). B) Mean infarct volume in the group of animals exposed to permanent MCAO for 24 hours and subjected to two dialysis cycles (the data is expressed as mean±SEM, n=4, ^{*}p>0.05 with respect to the control). t=0 indicates the start of the middle cerebral artery occlusion surgery.

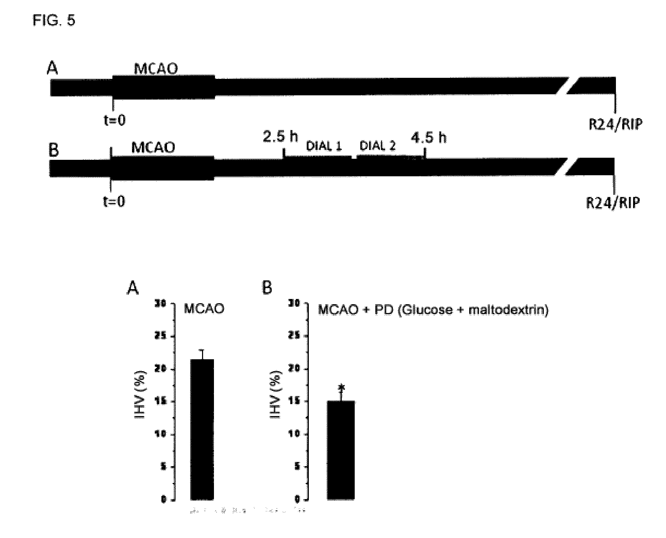

Figure 5 shows the effect of peritoneal dialysis with a dialysis solution prepared from the composition specified in Table 5 on infarct volume after middle cerebral artery occlusion (MCAO).
   A) Mean infarct volume in the group of animals exposed to permanent MCAO for 24 hours (the data is expressed as mean±SEM, n=6). B) Mean infarct volume in the group of animals exposed to permanent MCAO for 24 hours and subjected to two dialysis cycles (the data is expressed as mean±SEM, n=4, ^{*}p<0.05 with respect to the control). t=0 indicates the start of the middle cerebral artery occlusion surgery.

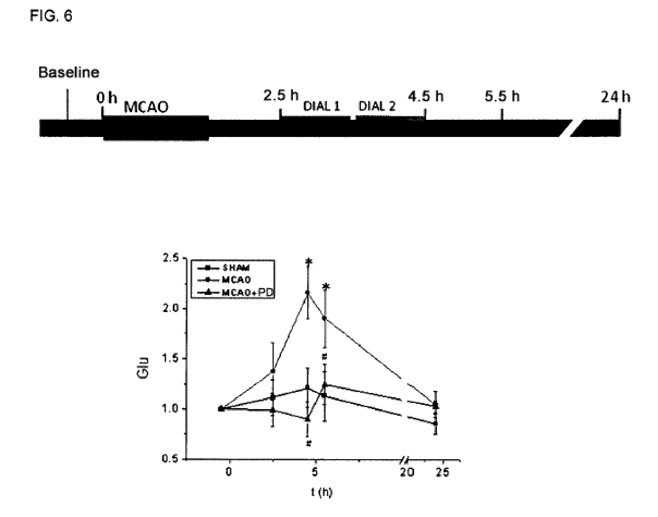

Figure 6 shows the effect of peritoneal dialysis with a dialysis solution prepared from the composition specified in Table 1 on plasma glutamate concentration after middle cerebral artery occlusion (MCAO).
   The samples were collected at different times indicated in the graph, as indicated in the diagram. Plasma glutamate values are normalized to their respective baseline values (obtained before surgery). (The data is expressed as mean±SEM, n=5 for the SHAM group; n=6 for the MCAO group, p<0.05 with respect to the baseline; n=5 for MCAO+PD -peritoneal dialysis-; ^{#}p<0.05 with respect to MCAO).

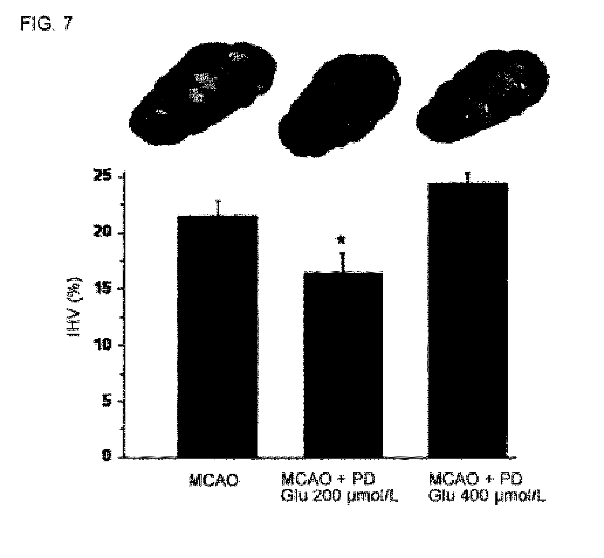

Figure 7 shows the effect of the addition of glutamate to the dialysis solution prepared from the composition specified in Table 1 on infarct volume after middle cerebral artery occlusion (MCAO).
   The rats were exposed to permanent MCAO for 24 hours (MCAO, n=6) and two groups of animals were subjected to two peritoneal dialysis cycles with a dialysis solution prepared from the composition specified in Table 1 to which 200 µM glutamate (200 µM MCAO+PD; n=6) or 400 µM glutamate (400 µM MCAO+PD; n=4) was added. ^{*}p<0.05 with respect to MCAO.

### Embodiment of the Invention

Different examples are shown below to illustrate the present invention without limiting the scope thereof.

### Example 1

### Test for evaluating if peritoneal dialysis with a dialysis solution prepared from a composition containing a physiological glucose concentration and buffered to pH 6.5 with phosphate significantly reduces infarct volume in infarction caused by middle cerebral artery occlusion

Healthy male Sprague-Dawley rats weighing 250-300 g were anaesthetized with 1.5% isoflurane in a mixture of 80% atmospheric air/20% oxygen. The body temperature of the animals was monitored throughout the entire experiment by means of a rectal probe and was kept at 36.5±0.5°C using a thermal blanket. Permanent focal cerebral ischemia was induced by distal middle cerebral artery (MCA) occlusion as described previously (Sobrado M, et al. Neuroscience 2003. 118:107-13). To ligate the MCA, a craniotomy was performed to expose the region of the trunk thereof. MCA occlusion was performed with a 9/0 nylon right before the split of the frontal and parietal branches. The common carotid arteries (CCAs) were then exposed, being occluded permanently with a 6/0 nylon in the case of ipsilateral and temporarily in the case of contralateral, eliminating the occlusion from the latter after 90 minutes. Rats in which the MCA was exposed but not occluded were used as controls with simulated intervention (SHAM, n=5).

After the surgical intervention, a group of animals (n=6) was returned to their cages and they had free access to food and water; another group (n=5) was subjected to two peritoneal dialysis cycles (lasting one hour each) with 40 mL/Kg of the dialysis solution prepared from the composition specified in Table 1 and sterilized by filtration through a 0.22 µm membrane filter, between 2.5 and 4.5 hours after surgery. The rats were sacrificed 24 hours after the surgical intervention to determine the infarct volume.

| Table 1. Composition | | |
|---|---|---|
| Compound | Concentration | mEq/L |
| Na⁺ | | 140 |
| Cl⁻ | | 140 |
| Ca²⁺ | | 3.5 |
| Mg²⁺ | | 0.0 |
| K⁺ | | 0.0 |
| Lactate | 3.5 mmol/L | |
| Phosphate | 10 mmol/L, pH=6.5 | |
| Glucose | 1.5 g/L | |

To determine infarct volume, the brain was extracted and 2 mm thick coronal slices (Brain Matrix, WPI, UK) were taken and stained with 2,3,5-triphenyltetrazolium chloride (1% TTC in 0.2 M phosphate buffer). The infarct volumes were calculated by obtaining images of the samples of each side of the coronal sections using a digital camera (Nikon Coolpix 990), and the images were analyzed using ImageJ 1.33u (National Institutes of Health, Bethesda, MD). The digitalized image was projected on a video monitor, the experimental conditions having been concealed from the observer. The perimeter of the contralateral hemisphere was superimposed on the ipsilateral hemisphere to exclude edema, and the margins of the infarction were outlined with a cursor. The unstained infarct area was determined by means of counting the pixels contained in the outlined regions. Infarct volumes (in % of infarcted hemisphere -IHV-) were obtained based on the integration of the infarct areas throughout the extension of the infarction calculated as an orthogonal projection. All the animals showed infarction after the occlusion process, which only affected the cerebral cortex.

No lesion was detected in the group of animals in which artery occlusion was not performed (SHAM). The middle cerebral artery occlusion produced a infarct volume, determined 24 hours after surgery, of 21.42±1.48%, n=8 (Figure 1). In the group of animals that were subjected to two peritoneal dialysis cycles with the solution obtained from the composition of Table 1, the infarct volume was significantly less (Figure 1), being 12.1± 2.1%, n=12 (p<0.001).

### Example 2

### Test for evaluating if peritoneal dialysis with a dialysis solution prepared from a composition containing a physiological glucose concentration and buffered to pH 6.5 with bicarbonate significantly reduces infarct volume in infarction caused by middle cerebral artery occlusion

A method similar to that described in Example 1 was carried out using the composition specified in Table 2 for preparing the sterile dialysis solution.

| Table 2. Composition | | |
|---|---|---|
| Compound | Concentration | mEq/L |
| Na⁺ | | 140 |
| Cl⁻ | | 140 |
| Ca²⁺ | | 3.5 |
| Mg²⁺ | | 0.0 |
| K⁺ | | 0.0 |
| Lactate | 15 mmol/L | |
| Phosphate | 25 mmol/L, pH=6.5 | |
| Glucose | 1.5 g/L | |

The determination of the infarct volume using bicarbonate as a pH buffer showed that there was no difference in infarct volumes between the control animals (21.42±1.48%, n=8) and the dialyzed group (18.95±3.01%, n=4; p>0.05, t-test) (Figure 2).

### Example 3

### Test for evaluating if peritoneal dialysis with a dialysis solution prepared from a hypertonic composition containing a very high glucose concentration and buffered to pH 6.5 with phosphate significantly reduces infarct volume in infarction caused by middle cerebral artery occlusion

A method similar to that described in Example 1 was carried out using the composition specified in Table 3 for preparing the sterile dialysis solution.

| Table 3. Composition | | |
|---|---|---|
| Compound | Concentration | mEq/L |
| Na⁺ | | 140 |
| Cl⁻ | | 140 |
| Ca²⁺ | | 3.5 |
| Mg²⁺ | | 0.0 |
| K⁺ | | 0.0 |
| Lactate | 3.5 mmol/L | |
| Phosphate | 10 mmol/L, pH=6.5 | |
| Glucose | 38 g/L | |

The determination of the infarct volume using a hypertonic composition with a very high glucose concentration showed that there were no differences in infarct volume between the control animals (21.42±1.48%, n=8) and the group of dialyzed animals (17.61±2.12%, n=4; p>0.05, t-test) (Figure 3).

### Example 4

### Test for evaluating if peritoneal dialysis with a dialysis solution prepared from an isotonic concentration not containing glucose and buffered to pH 6.5 with phosphate significantly reduces infarct volume in infarction caused by middle cerebral artery occlusion

A method similar to that described in Example 1 was carried out using the composition specified in Table 4 for preparing the sterile dialysis solution.

| Table 4. Composition | | |
|---|---|---|
| Compound | Concentration | mEq/L |
| Na⁺ | | 140 |
| Cl⁻ | | 140 |
| Ca²⁺ | | 3.5 |
| Mg²⁺ | | 0.0 |
| K⁺ | | 0.0 |
| Lactate | 3.5 mmol/L | |
| Phosphate | 10 mmol/L, pH=6.5 | |
| Glucose | 0 g/L | |
| Maltodextrin | 29.9 g/L | |

The determination of the infarct volume using a glucose-free isotonic composition showed that there were no differences in infarct volume between the control animals (21.42±1.48%, n=8) and the group of dialyzed animals (21.37±2.76%, n=4; p>0.05, t-test) (Figure 4).

### Example 5

### Test for evaluating if peritoneal dialysis with a dialysis solution prepared from an isotonic composition containing a low glucose concentration and buffered to pH 6.5 with phosphate significantly reduces infarct volume in infarction caused by middle cerebral artery occlusion

A method similar to that described in Example 1 was carried out using the composition specified in Table 5 for preparing the sterile dialysis solution.

| Table 5. Composition | | |
|---|---|---|
| Compound | Concentration | mEq/L |
| Na⁺ | | 140 |
| Cl⁻ | | 140 |
| Ca²⁺ | | 3.5 |
| Mg²⁺ | | 0.0 |
| K⁺ | | 0.0 |
| Lactate | 3.5 mmol/L | |
| Phosphate | 10 mmol/L, pH=6.5 | |
| Glucose | 0.75 g/L | |
| Maltodextrin | 14.95 g/L | |

The determination of the infarct volume using an isotonic composition with a low glucose concentration showed that there were differences in the infarct volume between the control animals (21.42±1.48%, n=8) and the group of dialyzed animals (15.19±2.2%, n=4; p<0.05, t-test) (Figure 5).

### Example 6

### Test for evaluating if middle cerebral artery occlusion causes an increase in plasma glutamate levels and if peritoneal dialysis with a dialysis solution prepared from an isotonic composition containing a physiological glucose concentration and buffered to pH 6.5 with phosphate reduces said levels

Healthy male Sprague-Dawley rats weighing 250-300 g were anaesthetized with 1.5% isoflurane in a mixture of 80% atmospheric air/20% oxygen. A cannula was inserted into the femoral artery for taking blood samples. The experiment continued as described in Example 1. The rats were divided into three groups, a group in which the MCA was exposed but not occluded served as controls with simulated intervention (SHAM, n=5), another group in which middle cerebral artery occlusion (MCAO, n=6) was performed, and another group in which middle cerebral artery occlusion was performed and the animals were subjected to two peritoneal dialysis cycles (MCAO+PD, n=5) with the dialysis solution prepared from the composition indicated in Table 1. After the surgical intervention and after taking the blood samples, the animals were returned to their cages and had free access to food and water. The rats were sacrificed 24 hours after the surgical intervention.

Plasma samples were obtained by means of centrifuging the blood sample (100 µl) at 4°C in the presence of 3.15% citrate buffer (10 µl) at 3,500 r.p.m. for 5 minutes. The plasma forming the top layer was removed with a Pasteur pipette and deposited in a tube which was stored at -80°C until processing.

Plasma glutamate was determined by means of an enzymatic assay described by Nicholls, DG et al. (J. Neurochem. 1987. 49: 50-57) by measuring the increase in fluorescence produced as NADPH is generated in the reaction catalyzed by the glutamate dehydrogenase (GDH) enzyme in the presence of NADP⁺.

The changes in fluorescence were registered in Perkin-Elmer LS-50B and LS-55 fluorimeters at emission and excitation wavelengths of 340 nm and 460 nm, respectively. The excitation and emission slits were fixed at 3 nm and 9 nm.

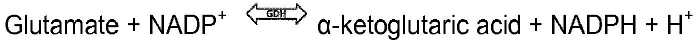

The determinations were performed in an HBM medium (140 mM NaCl, 5 mM KCI, 5 mM NaHCO₃, 1.2 mM NaH₂PO₄, 1 mM MgCl₂ and 10 mM HEPES; pH 7.4) containing 1 mM NADP⁺, 1.33 mM CaCl₂ and 50 units of glutamate dehydrogenase. The test was started by adding 5 µl of plasma. A standard of 2 nmoles of glutamate was added at the end of each determination for quantifying the content of the sample.

Middle cerebral artery occlusion caused a greater than 2-fold increase in plasma glutamate concentration after 4.5 hours of occlusion, as shown in Figure 6. Said increase in plasma glutamate was not observed in the rats from the SHAM group or in the group of dialyzed rats (Figure 6).

The results shown in Figure 6 confirm that plasma glutamate increases following cerebral ischemia and that peritoneal dialysis with a dialysis solution prepared from the composition of the invention is a very effective method for reducing it.

### Example 7

### Test for evaluating the effect of the presence of glutamate in the dialysis solution prepared from the composition of the invention on infarct volume after middle cerebral artery occlusion (MCAO).

Healthy male Sprague-Dawley rats weighing 250-300 g were anaesthetized with 1.5% isoflurane in a mixture of 80% atmospheric air/20% oxygen. The experiment continued in the manner described in Example 1. The rats were divided into three groups, a control group in which middle cerebral artery occlusion (MCAO, n=6) was performed, a group in which middle cerebral artery occlusion was performed and the animals were subjected to two peritoneal dialysis cycles with the dialysis solution prepared from the composition indicated in Table 1 to which a glutamate concentration of 200 µmol/L (200 µM MCAO+PD; n=6) was added, and a third group in which middle cerebral artery occlusion was performed and the animals were subjected to two peritoneal dialysis cycles with the dialysis solution prepared from the composition indicated in Table 1 to which a glutamate concentration of 400 µmol/L (400 µM MCAO+PD; n=4) was added. The rats were sacrificed after 24 hours to determine the infarct volume as explained in Example 1.

When performing peritoneal dialysis, all the substances that are not present in the dialysis solution are expected to be dialyzed. Therefore, to check if the reduction of the infarct volume was the result of the reduction of the plasma glutamate concentration and not of the concentration of another substance, glutamate was introduced in the composition of the invention at two different concentrations, 200 and 400 µM. As shown in Figure 7, when glutamate was present in the composition at a concentration of 200 µM, the infarct volume still dropped significantly in relation to that found in the control rats (MCAO). When the glutamate concentration in the composition was increased to 400 µM, the infarct volume was similar to that of the control ischemic rats (24.40±0.91 %).

According to the results shown in Figure 7, when the peritoneal dialysis solution contains glutamate at a concentration of 200 µmol/L, it reduces the solution's capacity for reducing the infarct volume, and the peritoneal dialysis solution is completely ineffective when the glutamate concentration is 400 µmol/L.

## Claims

1. Use of an isotonic composition buffered to a physiologically acceptable pH in the preparation of a dialysis solution for the treatment of a cerebrovascular disease by means of peritoneal dialysis.

2. Use according to claim 1, wherein the composition comprises electrolyte concentrations of 110 to 150 mEq/L of sodium ion, of 0 to 5 mEq/L of potassium ion, of 0 to 2 mEq/L of magnesium ion, of 0 to 6 mEq of calcium ion and of 80 to 150 mEq/L of chloride ion.

3. Use according to any of claims 1-2, where the pH is between 6 and 7.5 and it is buffered by means of a mixture of lactic acid and phosphate.

4. Use according to claim 3, wherein the lactic acid concentration is 0 to 3.5 mmol/L.

5. Use according to claim 4, wherein the phosphate concentration is 10 mmol/L.

6. Use according to any of the preceding claims, wherein the composition contains a glucose concentration of between 0.5 and 2.0 g/L.

7. Use according to claim 6, wherein the glucose concentration is 0.75 g/L.

8. Use according to claim 6, wherein the glucose concentration is 1.5 g/L.

9. Use according to any of the preceding claims, wherein the composition contains a glutamate concentration equal to or less than 200 µM.

10. Use according to any of the preceding claims, wherein the composition contains dextrin.

11. Use according to any of the preceding claims, where the cerebrovascular disease is cerebral ischemia.

12. Use according to claim 9, where the cerebral ischemia is acute or chronic cerebral ischemia and is one of the following diseases: transient ischemic attack, traumatic brain injury, cerebral ischemic stroke or cerebral hemorrhagic stroke.
